# EUROPÄISCHE PATENTSCHRIFT

(11) **EP 2 449 358 B1**
(45) Veröffentlichungstag und Bekanntmachung des Hinweises auf die Patenterteilung: **29.05.2013**
(21) Anmeldenummer: 10744661.9
(22) Anmeldetag: 30.06.2010
(51) Int. Cl.: G01N 1/12, G01N 1/18

(54) **VORRICHTUNG ZUR WASSERPROBENNAHME**
WATER SAMPLING DEVICE
DISPOSITIF SERVANT À PRELEVER DES ECHANTILLONS D'EAU

(30) Priorität: 03.07.2009 DE 102009032097
(43) Veröffentlichungstag der Anmeldung: 09.05.2012
(73) Patentinhaber: Stiftung Alfred-Wegener-Institut für Polar- und Meeresforschung, 27570 Bremerhaven (DE)
(72) Erfinder: WULFF, Thorben, 68219 Mannheim (DE); SAUTER, Eberhard, J., 27721 Ritterhude (DE)
(86) Internationale Anmeldenummer: PCT/DE2010/000770
(87) Internationale Veröffentlichungsnummer: WO 2011/000364

(56) Entgegenhaltungen:
- DE-A1- 2 221 377
- DE-A1- 4 132 410
- US-A- 3 489 012
- US-A- 5 138 890

## Beschreibung

Die Erfindung bezieht sich auf eine Vorrichtung zur Wasserprobennahme mit einem Montagegestell, einem Mehrfachmagazin in Form eines Trommelmagazins mit mehreren Probenbehältern, die durch individuelle Befestigungseinrichtungen einzeln entnehmbar sind, wobei formschlüssige Steckverbindungen zur Befestigung der Probenbehälter mit dem Trommelmagazin vorgesehen sind, und an einer ersten Stirnseite ein Einlassventil und an einer zweiten Stirnseite ein Auslassventil aufweisen, wobei axial angeordnete konische Öffnungen in den Stirnseiten der Probenbehälter und dazu formschlüssige Verschlusskegel als Einlass- und Auslassventile vorgesehen sind, mit einer Betätigungsvorrichtung zum Verschließen der Einlass- und Auslassventile und einem Motor mit Getriebe als Anwahleinrichtung für den nächsten zur Wasserprobennahme bestimmten Probenbehälter.

Derartige Vorrichtungen zur Wasserprobennahme ermöglichen von beliebigen Wasserfahrzeugen aus die Entnahme hochgenau bestimmbarer Wasserproben aus Gewässern jeder Art und Tiefe und deren Rückführung zur Oberfläche. Die Wasserproben werden dabei einzeln in separate und abdichtbare Probenbehälter genommen, die später auch einzeln der Vorrichtung zur Wasserprobennahme entnommen und einer Analyse zugeführt werden können. Bei der Wasserprobennahme kann es sich um die Aufnahme von Wasserprofilen in senkrechter Anordnung, um zeitlich versetzte Wasserproben desselben Ortes oder um beliebige Probenfolgen handeln. Zur Wasserprobennahme wird der jeweilige Probenbehälter an beiden Stirnseiten durch Öffnen der Einlass- und Auslassventile bereit gemacht. Der offene Probenbehälter wird nun durch Bewegung der ganzen Vorrichtung zur Wasserprobennahme oder durch eine am Ort der Wasserprobennahme vorhandene Strömung durchspült. Nach Ablauf einer frei wählbaren Zeit wird der Probenbehälter durch ein Triggersignal an die Betätigungsvorrichtung der Einlass- und Auslassventile geschlossen. Im Inneren befindet sich nun die gewünschte Wasserprobe in genau dem am Probenort herrschenden Wasserzustand. Anschließend kann der nächste Probenort oder die nächste Probenzeit zusammen mit dem nächsten Probenbehälter gewählt werden. Bei Probennahmen in großen Wassertiefen mit entsprechendem Wasserdruck wird beim Zurückführen der Vorrichtung zur Wasserprobennahme an die Wasseroberfläche die Druckdifferenz zu einer Abgabe einer sehr geringen Wassermenge durch die Dichtungen der Einlass- und Auslassventile führen. Da nur Wasser abgegeben und keines aufgenommen werden kann, kommt es zu keiner Verfälschung der Zusammensetzung der Wasserprobe.

### STAND DER TECHNIK

Aus der JP 58044326 A ist eine Vorrichtung zur Wasserprobennahme mit einer Mehrzahl von Probenbehältern bekannt. Durch einen komplizierten elektromechanischen Mechanismus werden die Probenflaschen geöffnet und nach Befüllung wieder geschlossen. Die Probenflaschen haben nur eine Öffnung, ein Durchspülen ist nicht vorgesehen. Jede Flasche weist ihren eigenen aufwändigen elektromechanischen Betätigungsmechanismus auf, der durch einen zentralen Exzenter mit einem Motorantrieb betätigt wird. Aus der JP 01084131 A ist eine Vorrichtung zur Wasserprobennahme bekannt, die die Proben in einem gemeinsamen Magazin speichert. Ein Wasserrad, das zum Hauptanteil oberhalb der Wasseroberfläche gehalten wird, schöpft das Probenwasser mittels-tassenähnlicher Schöpfer und leitet es in einen Behälter, von dem aus es in eine der Kammern des Magazins befördert wird. Diese Vorrichtung ist zum Einsatz unter der Wasseroberfläche nicht geeignet, die Anwahl des jeweiligen Probenbehälters im Magazin ist aufwändig. Aus der US 5,441,071 ist eine Vorrichtung zur Wasserprobennahme bekannt, die eine Vielzahl von Probenkammern aufweist. Jede Probenkammer hat ihr eigenes Einlassventil. Alle Ausgänge sind miteinander verbunden und eine in beiden Richtungen wirkende Pumpe befördert in der einen Richtung das Probenwasser in einen Sammelbehälter und in der andern Richtung eine Reingungsflüssigkeit in die zuvor benutzte Probenkammer. Die Reinigungsflüssigkeit wird vor dem nächsten Einsatz wieder ausgespült. Das Gerät ist für den Einsatz in Schmutzwasserumgebungen konzipiert, die Notwendigkeit der Spülung mit Reinigungsflüssigkeit kompliziert den Prozess und erhöht den apparativen Aufwand. Aus der DE 40 12 625 C2 ist eine Vorrichtung zur Wasserprobennahme zum speziellen Einsatz in Bohrlöchern bekannt, die keine Wechselvorrichtung aufweist und daher nur eine einzelne Probe nehmen kann. Auch hier dient ein elektromechanischer Mechanismus zum Öffnen und Schließen der einzigen Öffnung zum Befüllen des Probenbehälters. Sensoren erfassen bestimmte Parameterwerte des z.B. in einem Bohrloch befindlichen Wassers und melden diese an eine Steuereinheit, die bei Erreichen bestimmter Werte die Wasserprobennahme auslöst. Auch aus der AT 398 002 B ist eine Vorrichtung zur Wasserprobennahme zum Einsatz insbesondere in Brunnen und Bohrlöchern bekannt, die keine Wechselvorrichtung aufweist und daher nur eine einzelne Probe nehmen kann. Der Probenbehälter weist zwei durch eine Schnur verbundene Ventilklappen auf. Beim Absenken des Probenbehälters im Wasser wird die untere Ventilklappe durch den Strömungsdruck offen gehalten, die obere Ventilklappe wird durch einen Haltedauermagneten offen gehalten. Nach Erreichen der gewünschten Tiefe wird die untere Ventilklappe durch Schwerkraft in Verschlussrichtung bewegt. Endgültig verschlossen wird der Probenbehälter durch einen Ruck am Halteseil, durch den die obere Ventilklappe vom Haltedauermagneten gelöst und beide Ventilklappen in die Verschlussstellung gebracht werden. Der Ventilmechanismus ist sehr einfach und kann wegen der Auslösung durch Seilzug in größeren Wassertiefen nicht mehr eingesetzt werden. Aus der EP 1 493 656 A1 ist eine Vorrichtung zur Wasserprobennahme bekannt, die als Nutzlast von einem Unterseeboot getragen wird. Es ist vorgesehen, eine Mehrzahl von Proben zu nehmen und in getrennten Probenbehältern zu separieren. Eine Spüleinrichtung für die Einlassrohrleitung soll sicherstellen, dass nur das jeweils gewünschte Wasser in den nächsten Probenbehälter gelangt. Die Formen von Verschlüssen und Betätigungseinrichtungen werden in dieser Druckschrift nicht näher beschrieben. Aus der DE 102 32 623 B4 ist eine Vorrichtung zur Wasserprobennahme bekannt, die mit einer Mehrzahl von waagerecht übereinander angeordneten Probenbehältern in einem Absetzgestell ein Profil von Bodenwasser in beliebigen Gewässern nehmen kann, indem alle Probenbehälter gleichzeitig geöffnet und nach Beruhigung des umgebenden Wassers auch gleichzeitig wieder geschlossen werden. Die Inhalte der Probenbehälter repräsentieren die Wasserschichten in unmittelbarer Bodennähe. Die Probenbehälter werden mit Verschlussstopfen nach Art der bekannten Niskin-Flaschen beidseitig verschlossen. Die mittels eines rückstellenden Gummizugs verbundenen Verschlussstopfen werden zu Beginn des Einsatzes geöffnet und mittels eines äußerlichen Korrosionsdrahtes offen gehalten. Nach der gleichzeitigen Wasserprobennahme in allen Behältern wird das Schließen der Behälter ausgelöst, indem ein Stromfluss den Korrosionsdraht im Seewasser schnell durchkorrodiert, so dass alle Probenbehälter durch ihre Verschlussstopfen mit den rückstellenden Gummizügen gleichzeitig dicht verschlossen werden. Eine individuelle Wasserprobennahme je Probenbehälter ist mit dieser Vorrichtung aufgrund ihrer Konstruktion nicht möglich.

Aus der Veröffentlichung I (EXOCET D, WP5; Compilation of instrument specifications for integration on the ROV of the IFREMER VICTOR 6000 for the MOMARETO cruise, Seiten 8 und 9; www.ifremer.fr/exocetd/documents/- results/5D1.pdf; gefunden am 05.06.2009) ist eine Vorrichtung zur Wasserprobennahme "Pepito" bekannt, die fünfundzwanzig Probenbehälter aufweist, die durch ein Ventilsystem in dem Aufbaugehäuse gefüllt werden können. Eine Ventilsteuerung wird in der Veröffentlichung nicht beschrieben. Die Vorrichtung zur Wasserprobennahme ist als Last für ein unabhängig bewegbares Unterwasserfahrzeug vorgesehen und wird über einen Rechner gesteuert. Eine Durchströmung der Probenbehälter ist nicht vorgesehen. Aus der **Veröffentlichung II** (Development of an active, large volume, discrete seawater sampler for autonomous underwater vehicles; www.mbari.org/staff/ryjo/pdfs/Bird_et_al 2007.pdf; gefunden am 05.06.2009) ist eine Vorrichtung zur Wasserprobennahme "Gulper" bekannt, die auf einem AUV (Autonomous Underwater Vehicle) eingesetzt wird und zehn 2-Liter-Probenbehälter aufweist. Jeder Probenbehälter besteht aus einem zylinderförmigen Gehäuse, einem Einweg-Einlassventil und einem Kolben im Inneren, der zum Zeitpunkt der Wasserprobennahme durch eine gespannte Feder aufgezogen wird und die Wasserprobe in weniger als zwei Sekunden einsaugt. Die gespannte Feder wird über eine Klinke festgehalten. Eine Auslöseleine kann im Bedarfsfall gezogen werden, wodurch die Klinke entfernt und die Feder ausgelöst wird. Die Vorrichtung ist wegen der Seilzugauslösung für einen von dem Wasserfahrzeug unabhängigen Einsatz in großen Tiefen ungeeignet.

Aus der DE 41 32 410 A1 ist ein Probennehmer bekannt, deren Probenbehälter nach Art von Niskin-Flaschen oben und unten durch klappbare Deckel verschließbar sind. Die Deckel werden hier durch eine außen liegende Feder und einen Auslösemechanismus mit Reißleine betätigt. Der Probennehmer vermeidet den Nachteil eines im Inneren verlaufenden Gummibandes, das gegebenenfalls die eingeschlossene Probe kontaminieren und damit ein Messergebnis verfälschen kann.

Aus der WO 99/18421 A1 ist ein Probennehmer bekannt, der automatisch Wasserproben über einen Filter einsaugen, das Filtrat chemisch-biologisch behandeln und nach einer Analyse die verwendeten Filterscheiben in Probenröhren sammeln kann. Die Probenröhren sind unten permanent geschlossen und oben nur zur Einlagerung einer weiteren Filterscheibe durch Drehen eines Filterkarussells kurzfristig öffenbar.

Aus der US 7,178,415 B2 ist ein Probenbehälter bekannt, der ähnlich einer Niskin-Flasche eine im Inneren angeordnete Feder zum Verschluß beider Enden mit klappbaren Deckeln aufweist, die von einer Auslösevorrichtung betätigt werden. Der Probenbehälter ist handlich und die Deckel schließen fest, sodass die Proben im Probenbehälter verbleiben können, bis eine Analyse vorgenommen werden kann, ein Umfüllen ist nicht nötig. Ein Probenwechsler ist nicht vorgesehen.

Aus der US 5,341,693 A ist ein fester Probenbehälter mit einem flexiblen zweiten Innenbehälter bekannt, der in vorbekannter Weise durch außenliegende Federelemente geöffnet und geschlossen werden kann. Der Innenbehälter vermeidet durch sein Material die Kontamination der Probe durch das Material des festen Probenbehälters. Im Raum zwischen den Behältern kann Luft oder Wasser zur Temperaturregulation eingefüllt werden. Bei der Entnahme der Probe aus dem flexiblen Innenbehälter kann der Kontakt mit Luft auf ein Minimum beschränkt werden.

Aus der US 5,303,600 A ist ein flexibler Probenbehälter bekannt, der im entleerten Zustand zusammengefaltet ist und keine Auftrieb verursachende Luft beim Ablassen in die vorgesehene Tiefe enthält. Ein zusätzliches Abtriebsgewicht wird vermieden. Ein außenliegender federbelasteter Mechanismus kann den Behälter über drehbar gelagerte Zylinderhähne öffnen und schließen.

Aus der US 5,138,890 A ist ein Auslösemechanismus für den Verschluss von Probenbehältern an einem Vielfach-Probennehmer bekannt, der auf einer zentralen Achse im Zentrum des Probennehmers drehbar gelagert ist und über federnd gelagerte Stifte und Kugeln jeweils einen frei wählbaren Probenbehälter mittels der Freigabe einer Halteleine und damit verbundener Federelemente und Deckel an dem gewählten Probenbehälter verschließen kann.

Aus der US 3,489,012 A ist ist ein Auslösemechanismus für den Verschluss von Probenbehältern an einem Vielfach-Probennehmer bekannt, der auf einer zentralen Achse im Zentrum des Probennehmers drehbar gelagert ist und über federnd gelagerte Stifte jeweils einen nächsten in der Reihenfolge der Anordnung um die Achse befestigten Probenbehälter mittels der Freigabe einer Halteleine und damit verbundener Federelemente und Deckel verschließen kann.

Aus der JP 10 197 419 A ist ein Probenbehälter bekannt, der über an den inneren Enden befindliche konische Ventilsitze, Ventilkolben und Ventilfedern durch Aufbringen einer äußeren mechanischen Kraft entlang der Mittelachse nach innen geöffnet und bei Entlastung geschlossen werden können. Der Probenbehälter verfügt über ein die Außenwand durchgreifendes flexibles Element, dass für Druckausgleich in unterschiedlichen Wassertiefen sorgt, ohne die Proben zu kontaminieren.

Aus der US 4,852,413 ist eine Vorrichtung zur Wasserprobennahme mit einer Mehrzahl von Probenbehältern bekannt, die in Form eines Trommelmagazins, in der Druckschrift als Rosette bezeichnet, mit individuellen Befestigungsvorrichtungen außen an einem Montagegestell fest angeordnet und einzeln entnehmbar sind. Jeder Probenbehälter ist mit je einem eigenen Einlass- und einem Auslassventil ausgerüstet, wobei das Einlassventil von einer ebenfalls individuellen, sehr komplexen elektromechanischen Betätigungsvorrichtung betätigbar ist und das Auslassventil über einen Seiltrieb von Einlassventil synchron mitgenommen wird. Jeder Auslösemechanismus verfügt über eine gespannte Torsionsfeder, die ihren Probenbehälter genau einmal öffnen und wieder schließen kann. Die Auslösung der Öffnungs- und Schließvorgänge des nächsten, zur Wasserprobennahme bestimmten Probenbehälters kann über eine Anwahleinrichtung in Form eines Elektromagneten erfolgen, der durch einen Bediener oder mittels eines Programms triggerbar ist. Die Kraft des Auslösemechanismus wird durch einen steckbaren Sechskantantrieb auf das Einlassventil geleitet, so dass die Entnahme des Probenbehälters vom Montagegestell einfach möglich ist. Das Montagegestell wird an einem Tragseil hängend senkrecht durch die Wasserschichten bewegt und durch individuelles Öffnen und Schließen der Probenbehälter können Wasserproben an beliebigen Orten genommen werden. Ist ein Probenbehälter einmal mit einer Wasserprobe gefüllt und geschlossen, kann er erst nach Entnahme der Probe an Bord und erneutem Spannen der Torsionsfeder wieder eingesetzt werden. Die Probenbehälter haben einen länglich ovalen Querschnitt mit abgeflachten Seiten zur Platz sparenden Anordnung im Trommelmagazin. Die Einlass- und Auslassventile sind lang gestreckte Öffnungen an den oberen und unteren Stirnseiten der Probenbehälter, die von in darunter liegenden Ventilkörpern angeordneten, wasserdicht drehbar gelagerten Ventilzylindern geöffnet oder geschlossen werden. Die Ventilzylinder werden von der Betätigungseinrichtung über den Sechskantantrieb betätigt. Die Betätigungseinrichtung ist geprägt durch ein aufwändiges Zusammenspiel des Auslösemagneten mit einem Triggermechanismus aus zwei beweglich verbundenen Wellen mit Abflachungen für den Magnetstößel und Anschlagflächen einerseits und einem Spiralfortsatz andererseits in Verbindung mit dem Sechskantantrieb, einem Mitnehmerstift und der Torsionsfeder. Ventile und Betätigungseinrichtung sind sehr aufwändige und damit teure, empfindliche und wartungsintensive Konstruktionen.

Der der Erfindung nächstliegende Stand der Technik wird in der US 3 489 012 A offenbart. Bei der gattungsgemäßen Vorrichtung zur Wasserprobennahme weist das Montagegestell ein als Trommelmagazin ausgebildetes Mehrfachmagazin auf, das mehrere vertikal angeordnete Probenbehälter trägt. Diese sind durch formschlüssige Steckverbindungen als individuelle Befestigungseinrichtungen einzeln entnehmbar. An ihren beiden Stirnseiten sind die Probenbehälter frei zugänglich im Montagegestell angeordnet. Platten schließen das Montagegestell innerhalb der Probenbehälter nach oben und unten ab. Die Probenbehälter weisen an ihren beiden Stirnseiten konische Öffnungen und dazu formschlüssige Verschlusskegel als Ein- und Auslassventile auf, die über eine Betätigungsvorrichtung verschlossen werden können. Dabei werden die Verschlusskegel seitlich verkippt, sodass im geöffneten Zustand keilförmige Einfüllöffnungen für die Probenbehälter gebildet werden. Die bekannte Vorrichtung ist seitlich an einem Tragseil festgeklemmt, das zur Vermeidung von Verdrillung am Gewässerboden gegründet ist, sodass eine Drehung der Vorrichtung um das Tragseil herum nicht möglich ist oder allenfalls zu einer geringen Pendelbewegung führt. Ebenso ist zur Auswahl einzelner Probenbehälter keine Relativbewegung zwischen dem Trommelmagazin und dem Montagegestell vorgesehen. Vielmehr werden die einzelnen Probenbehälter durch eine umlaufende Nocke nacheinander verschlossen. Der Umlauf und die Position der Nocke werden über einen Motor als Auswahleinrichtung, der im Inneren der oberen Platte angeordnet ist, gesteuert. Durch die umlaufende Nocke und die weitgehend ortsfeste Positionierung der einzelnen Probenbehälter in Bezug auf den Wasserkörper wird jedem Probenbehälter jedoch ein anderer Ort der Probennahme zugeordnet.

### AUFGABENSTELLUNG

Die **AUFGABE** für die vorliegende Erfindung ist darin zu sehen, die zuvor genannte gattungsgemäße Vorrichtung zur Wasserprobennahme so weiterzubilden, dass jedem Probenbehälter derselbe Ort der Probennahme zugeordnet wird und nur eine Einfüllöffnung zur Befüllung sämtlicher Probenbehälter vorgesehen ist. Weiterhin soll eine Probennahme mit horizontal ausgerichteten Probenbehältern ermöglicht werden. Schließlich soll die Ausbildung der Einlass- und Auslassventile, die Betätigungseinrichtung für die Einlass- und Auslassventile und die Anwahleinrichtung für den nächsten zur Wasserprobennahme bestimmtem Probenbehälter derart vereinfacht werden, dass die Kosten für den Bau, das Gewicht, der Wartungsaufwand und die Zuverlässigkeit der Vorrichtung zur Wasserprobennahme entscheidend verbessert werden. Die erfindungsgemäße LÖSUNG für diesen Aufgabenkomplex ist dem Hauptanspruch zu entnehmen. Vorteilhafte Modifikationen der Erfindung sind den Unteransprüchen zu entnehmen, die im Nachfolgenden im Zusammenhang mit der Erfindung näher erläutert werden.

Bei der erfindungsgemäßen Vorrichtung zur Wasserprobennahme weist das Montagegestell weist zwei parallel zueinander angeordnete Stirnplatten auf, zwischen denen das Trommelmagazin um seine Mittelachse horizontal drehbar gelagert ist. Die Probenbehälter sind somit horizontal angeordnet und mit kraftschlüssigen Einspannverbindungen zwischen den Stirnplatten des Montagegestells eingespannt. Bei geöffneten Ein- und Auslassventilen an den Stirnseiten jedes Probenbehälters kann Wasser die Probenbehälter frei durchströmen. Nach der Wasserprobennahme werden die Ein- und Auslassventile geschlossen und die Wasserprobe sicher im Inneren eingeschlossen. Die Verschlusskegel der Einlass- und Auslassventile sind axial zu den Probenbehältern verschiebbar und bilden in nach außen verschobener Stellung zusammen mit der zugehörigen konischen Öffnung als ihrem Ventilsitz, das heißt im geöffneten Zustand der Ein- und Auslassventile, symmetrische Ringspalte. In nach innen verschobener Stellung dichten die Verschlusskegel durch eine formschlüssige Verbindung mit der zugehörigen konischen Öffnung als ihrem Ventilsitz, das heißt im geschlossenen Zustand der Ein- und Auslassventile, die Probenbehälter hermetisch ab. Mitnehmerhaken an den Verschlusskegeln und Auslösehebel in den Stirnplatten des Montagegestells bilden die Betätigungsvorrichtung zum Schließen der Ein- und Auslassventile der Probenbehälter. Der Motor mit Getriebe ist zwischen dem Montagegestell und dem Trommelmagazin montiert und dient der Drehung des Trommelmagazins um seine Mittelachse zwischen den Stirnplatten des Montagegestells. Dabei weist die eine Stirnplatte eine Einlassöffnung und die andere Stirnplatte eine Auslassöffnung auf. Zwischen diese ist ein Probenbehälter im Trommelmagazin zum Befüllen mit Wasser eindrehbar. Jeder Probenbehälter wird somit an demselben Ort mit Wasser befüllt.

Eine erste vorteilhafte Weiterbildung der erfindungsgemäßen Vorrichtung zur Wasserprobennahme wird durch zylinderförmig ausgebildete Probenbehälter erreicht. Durch die Zylinderform kann zur Herstellung der Probenbehälter ein einfaches Halbzeug verwendet werden, das in vielen Materialien und Durchmessern zur Verfügung steht oder leicht herstellbar ist. Eine für die Verteilung an einem Kreisumfang angepasste Querschnittsform der Probenbehälter ist durch individuelle Herstellung besonders kostenaufwändig und wird hier vermieden.

Durch zumindest je eine Passbohrung an Endplatten des Trommelmagazins und zumindest einen, jeder Passbohrung zugeordneten Stift an den Probenbehältern als formschlüssige Steckverbindungen der Befestigungseinrichtung der Probenbehälter mit dem Trommelmagazin und eine an der Auslassseite der Probenbehälter verschiebbar gelagerte federbelastete Hülse mit Bajonettverriegelung als kraftschlüssige Einspannverbindung der Befestigungseinrichtung der Probenbehälter zwischen den Stirnplatten des Montagegestells werden weitere vorteilhafte Weiterbildungen der erfindungsgemäßen Vorrichtung zur Wasserprobennahme erzielt. Für jeden Probenbehälter weisen die Endplatten des Trommelmagazins zumindest je eine Passbohrung auf, in die je ein an den Probenbehältern fixierter Passstift formschlüssig eingreift. Die Passstifte sind in einer Richtung angeordnet, so dass die Probenbehälter durch einfaches Einschieben leicht an dem Trommelmagazin aufgenommen werden können. Zusätzlich werden die Probenbehälter zwischen den Stirnplatten des Montagegestells kraftschlüssig eingespannt, indem die verschiebbar gelagerte federbelastete Hülse an der Auslassseite des Probenbehälters für den entsprechenden Anpressdruck sorgt. Zum Einsetzen eines Probenbehälters in das Trommelmagazin wird die verschiebbare Hülse gegen die Federkraft geschoben und ihre Bajonettverriegelung in eine Verriegelungsstellung gedreht. Der Probenbehälter ist damit ausreichend kurz, um zwischen den Stirnplatten des Montagegestells mit seinen Stiften in die Passbohrungen in den Endplatten des Trommelmagazins einschiebbar zu sein. Nach dem formschlüssigen Einschieben zur Fixierung am Trommelmagazin wird die Bajonettverriegelung der verschiebbaren Hülse aus ihrer Verriegelungsstellung gelöst, die Federkraft schiebt die Hülse weg und der Probenbehälter wird soweit verlängert, dass er nun zwischen den Stirnplatten des Montagegestells eingespannt wird.

Vorteilhaft kann Polyvinylchlorid als Material der federbelasteten Hülse eingesetzt werden. Polyvinylchlorid (PVC) ist leicht, seewasser- und im erforderlichen Bereich temperaturbeständig, kostengünstig, ausreichend formbeständig, um die Anforderungen an die Belastungen durch die Federkraft zu erfüllen und ist im gegensatz zu Polyethylen mit bekannten Klebemitteln sehr einfach weiter verarbeitbar. Als weitere vorteilhafte Weiterbildungen der erfindungsgemäßen Vorrichtung zur Wasserprobennahme kann Polytetrafluorethylen als Beschichtungsmaterial der Stirnplatten des Montagegestells eingesetzt werden und die Probenbehälter können an der Einlassseite eine umlaufende Nut mit einem um 0,1 bis 2 mm hervorstehend eingelegten Gleitring aus Polytetrafluorethylen aufweisen. Durch die Einspannung der Probenbehälter zwischen den Stirnplatten des Montagegestells wird beim Drehen des Trommelmagazins die Reibkraft zwischen den Stirnseiten der Probenbehälter und den Stirnplatten des Montagegestells durch die Verwendung von Polytetrafluorethylen (PTFE) für die Beschichtung der Stirnplatten und den Gleitring an der Einlassseite und durch den Einsatz von PVC für die Hülse an der Auslassseite des Probenbehälters so gering wie möglich gehalten.

Weiterhin können die Endplatten des Trommelmagazins mit kreisabschnittförmigen Aufnahmen in symmetrisch am Umfang verteilter Anordnung am Außenrand zur Aufnahme der zylinderförmigen Probenbehälter versehen sein. Durch eine derartige Formgebung wird die formschlüssige Fixierung der Probenbehälter am Trommelmagazin unterstützt.

Eine andere vorteilhafte Weiterbildung der erfindungsgemäßen Vorrichtung zur Wasserprobennahme sind Mitnehmerhaken an den Verschlusskegeln mit bündig zu den Stirnseiten der Probenbehälter ausgerichteten Flächen. Die bündig ausgerichteten Flächen stellen Prallflächen für das einströmende Probenwasser dar, durch die ein Staudruck an der Einlassseite und ein gegenüber dem umliegenden Wasser höherer Innendruck im Probenbehälter erzeugt wird. Dadurch wird verhindert, dass durch unvermeidliche Undichtheiten an der Einspannstelle zwischen der Stirnplatte des Montagegestells und der Stirnseite des Probenbehälters auf der Einlassseite unerwünschtes Wasser während der Wasserprobennahme in den Probenbehälter eindringt. Die Aufnahme von nicht für die Probe vorgesehenem, möglicherweise durch Verunreinigungen kontaminiertem Wasser muss für eine authentische Wasserprobennahme unbedingt vermieden werden.

Eine Lagerung der Verschlusskegel mit konkaven Lagerschalen auf in den Probenbehältern fixierten Zylinderstiften in geöffnetem Zustand der Einlass- und Auslassventile, eine Verbindung der Verschlusskegel der Einlass- und Auslassventile durch eine im Inneren der Probenbehälter verlaufende Zugfeder, sowie Haltefedern an den Auslösehebeln in den Stirnplatten des Montagegestells, die die Auslösehebel in einer ersten Endlage zumindest bündig, bevorzugt aber hinter den Oberflächen der Stirnplatten zurückstehend und in einer zweiten Endlage um zumindest 1 mm aus den Stirnplatten hervorstehend halten, bilden weitere vorteilhafte Weiterbildungen der erfindungsgemäßen Vorrichtung zur Wasserprobennahme. Im Fall der Wasserprobennahme steht der jeweils vorgesehene Probenbehälter mit geöffneten Einlass- und Auslassventilen vor entsprechenden Öffnungen in den Stirnplatten des Montagegestells, durch die das Probenwasser geleitet wird. Nach erfolgreicher Wasserprobennahme wird das Trommelmagazin durch die Anwahleinrichtung weiter gedreht, bis der nächste Probenbehälter vor den Öffnungen zur Wasserprobennahme bereit steht. Beim Herausdrehen des mit der Wasserprobe gefüllten Probenbehälters aus der Stellung zur Wasserprobennahme greifen die um zumindest 1 mm aus den Stirnplatten hervorstehenden Auslösehebel in die Mitnehmerhaken der Verschlusskegel ein. Beim Weiterdrehen werden die Verschlusskegel, die mit ihren konkaven Lagerschalen auf in den Probenbehältern fixierten Zylinderstiften ruhen, aus ihrer Ruheposition gezogen und dabei die Lagerung aufgehoben. Die im Inneren der Probenbehälter verlaufende, die Verschlusskegel verbindende Zugfeder zieht die Verschlusskegel in die konischen Öffnungen in den Stirnseiten des Probenbehälters und dichtet den Probenbehälter vollständig ab und schließt damit die Wasserprobe ein. Schließlich werden beim Weiterdrehen des Trommelmagazins die hinteren Ränder der Stirnseiten des gefüllten und verschlossenen Probenbehälters nun die federnd gelagerten Auslösehebel in ihre Stellung bündig zu den Stirnplatten des Montagegestells zurückdrücken. Sobald die vorderen Ränder der Stirnseiten des nächsten Probenbehälters die Auslösehebel passiert haben, können diese wieder ihre um zumindest 1 mm aus den Stirnplatten hervorstehende Stellung einnehmen.

Eine andere vorteilhafte Weiterbildung der erfindungsgemäßen Vorrichtung zur Wasserprobennahme bilden Probenbehälter, die in der Stirnfläche der Auslassseite eine zwischen zwei Endstellungen umklappbare Strömungsfahne aufweisen. In einer Stellung des Probenbehälters vor der Wasserprobennahme ist die Strömungsfahne eingeklappt, durch die abschließende Stirnplatte des Montagegestells gehalten und von außen nicht sichtbar. Bei erfolgreicher Wasserprobennahme wird die Strömungsfahne, die vor der Öffnung der Stirnplatte nun freien Bewegungsraum hat, durch den Druck der Wasserströmung herausgeklappt und in dieser Stellung beim Weiterdrehen des Probenbehälters durch die erneut abschließende Stirnplatte des Montagegestells gehalten. Sie ist nun von außen sichtbar und dokumentiert eine erfolgreiche Wasserprobennahme. Sofern es bei der geplanten Wasserprobennahme in einem Probenbehälter zu keiner Strömung kommt, klappt die Strömungsfahne nicht aus und bleibt nach dem Weiterdrehen unsichtbar. Damit wird eine erfolglose Wasserprobennahme dokumentiert.

Weiterhin kann vorteilhaft Polyvinylidenfluorid als Material des Probenbehälters eingesetzt werden. Polyvinylidenfluorid (PVDF) ist ein gegenüber Seewasser vollkommen inertes Material und sorgt dafür, dass die eingeschlossene Wasserprobe chemisch unverändert zur Analyse gelangt. Ein gegebenenfalls zur Zuleitung des Probenwassers von außerhalb eines Wasserfahrzeugs verwendetes Rohrleitungssystem kann ebenfalls in PVDF zur sicheren Vermeidung von Kontaminationen ausgeführt sein. Ferner kann Polyethylen als Material des Montagegestells vorteilhaft eingesetzt werden. Polyethylen (PE) ist leicht, formstabil, einfach verarbeitbar und kostengünstig und kann als Konstruktionsmaterial für wissenschaftliche Lasten zur erforderlichen Gewichtsreduktion entscheidend beitragen.

Eine Weiterdrehbarkeit des Trommelmagazins um jeweils eine durch die symmetrische am Umfang verteilte Anordnung der Probenbehälter bestimmte Teilung bei jeder Betätigung durch die Anwahleinrichtung stellt eine andere vorteilhafte Weiterbildung der erfindungsgemäßen Vorrichtung zur Wasserprobennahme dar. Alle Probenbehälter werden mit Einlass- und Auslassventilen in geöffneter Stellung in das Trommelmagazin eingesetzt. Die Betätigungsvorrichtung mit den Auslösehebeln in den Stirnplatten des Montagegestells kann die Probenbehälter nur schließen. In der bisher beschriebenen Ausführung wird jeder an der Betätigungsvorrichtung vorbei geführte Probenbehälter automatisch geschlossen. Es ist daher erforderlich, die Anwahleinrichtung so zu steuern, dass bei jeder Betätigung das Trommelmagazin genau um eine durch die symmetrische Anordnung der Probenbehälter bestimmte Teilung weiter bewegt wird, so dass alle Probenbehälter zur Wasserprobennahme verwendet und nicht ohne Probe geschlossen werden. Die Steuerung für die Anwahleinrichtung ist batteriegetrieben und in einem Druckbehälter am Montagegestell untergebracht.

### AUSFÜHRUNGSBEISPIELE

Die Vorrichtung zur Wasserprobennahme nach der Erfindung wird nachfolgend anhand von Ausführungsbeispielen für einzelne Details in den schematischen **FIGUREN** näher erläutert. Dabei zeigt:
- **FIGUR 1**: eine erfindungsgemäße Vorrichtung zur Wasserprobennahme aus einem Montagegestell bestückt mit einem Trommelmagazin in einer perspektivischen Ansicht von unten,
- **FIGUR 2**: ein Trommelmagazin mit Probenbehältern in verschiedenen Zuständen in einer Seitenansicht,
- **FIGUR 3**: einen Auslösehebel in perspektivischer Ansicht,
- **FIGUR 4**: einen Verschlusskegel in perspektivischer Ansicht,
- **FIGUR 5 A-E**: eine Betätigungsvorrichtung mit einem Ablauf in fünf Stellungen in Seitenansicht und
- **FIGUR 6A-C**: eine Strömungsfahne mit einem Ablauf in drei Stellungen in Seitenansicht.

Bei einzelnen **FIGUREN** und/oder in der Beschreibung nicht erwähnte oder gezeigte Bezugszeichen sind den vorangehenden **FIGUREN** bzw. deren Beschreibung zu entnehmen.

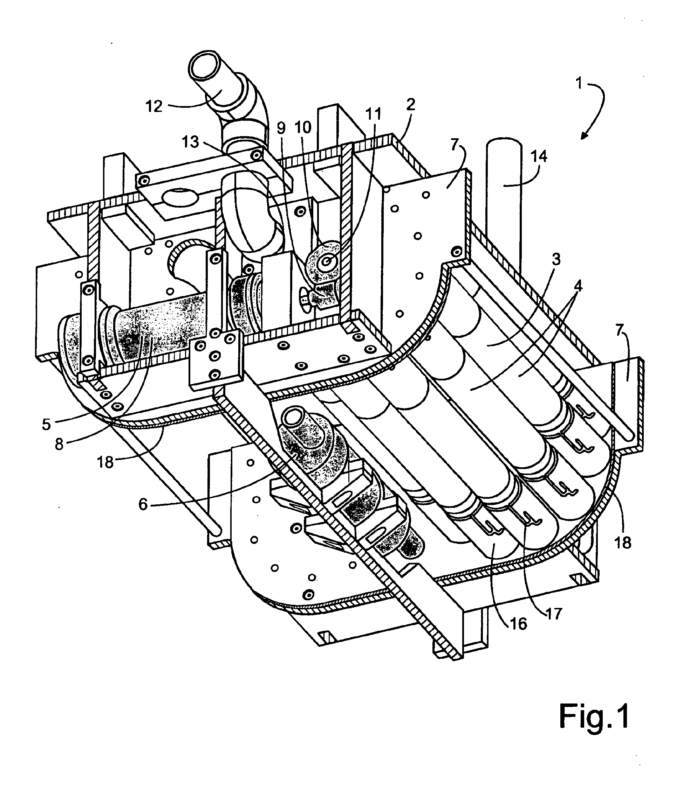

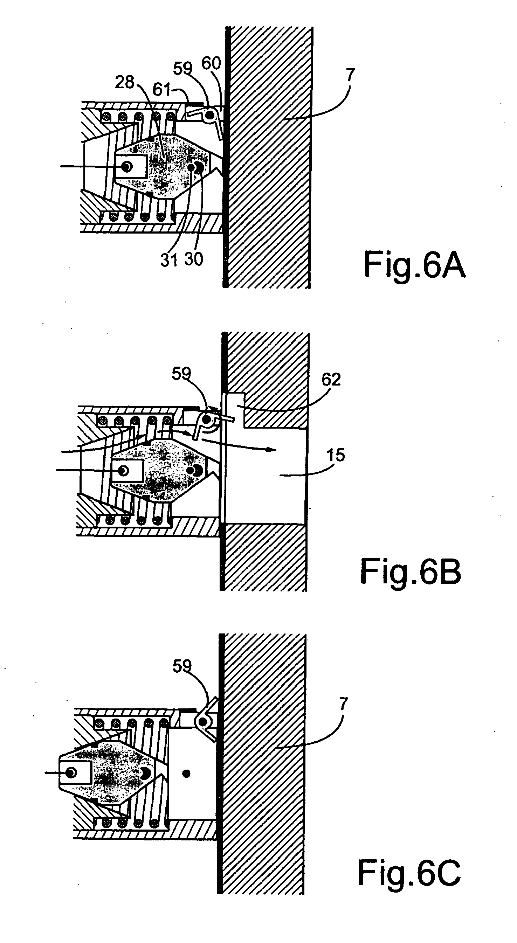

**FIGUR 1** zeigt eine erfindungsgemäße Vorrichtung zur Wasserprobennahme 1 aus einem Montagegestell **2**, hier bestückt mit einem von zwei möglichen Trommelmagazinen **3** in einer perspektivischen Ansicht von unten. Das Trommelmagazin **3** ist mit zylinderförmigen Probenbehältern **4** bestückt. Das Montagegestell **2** weist weiterhin eine Anwahleinrichtung **5** zur Drehung des Trommelmagazins **3** und eine Steuereinrichtung **6** zur Steuerung der Abläufe zur Wasserprobennahme auf. Das Trommelmagazin **3** ist drehbar zwischen zwei Stirnplatten **7** des Montagegestells **2** gelagert. Die Anwahleinrichtung **5** dreht das Trommelmagazin **3** mittels eines Motors **8** mit einer Schnecke **9** und einem Schneckenrad **10** auf einer zentralen Welle **11** des Trommelmagazins **3.** Eine Wasserströmung durch den zur Wasserprobennahme bereitstehenden Probenbehälter **4** wird durch eine Zuleitung **12** in einer Einlassöffnung **13** der ersten Stirnplatte **7** und eine Ableitung **14** in einer Auslassöffnung **15** (siehe **Figur 6B**) in der zweiten Stirnplatte **7** des Montagegestells **2** ermöglicht. Die Probenbehälter **4** des Trommelmagazins **3** sind zwischen den Stirnplatten **7** des Montagegestells **2** durch verschiebbar gelagerte federbelastete Hülsen **16** mit Bajonettverriegelung **17** eingespannt, wobei zur Verringerung der Reibung zwischen den Stirnseiten der Probenbehälter **4** und den Stirnplatten **7** des Montaggestells **2** die Stirnplatten **7** PTFE-Beläge **18** aufweisen.

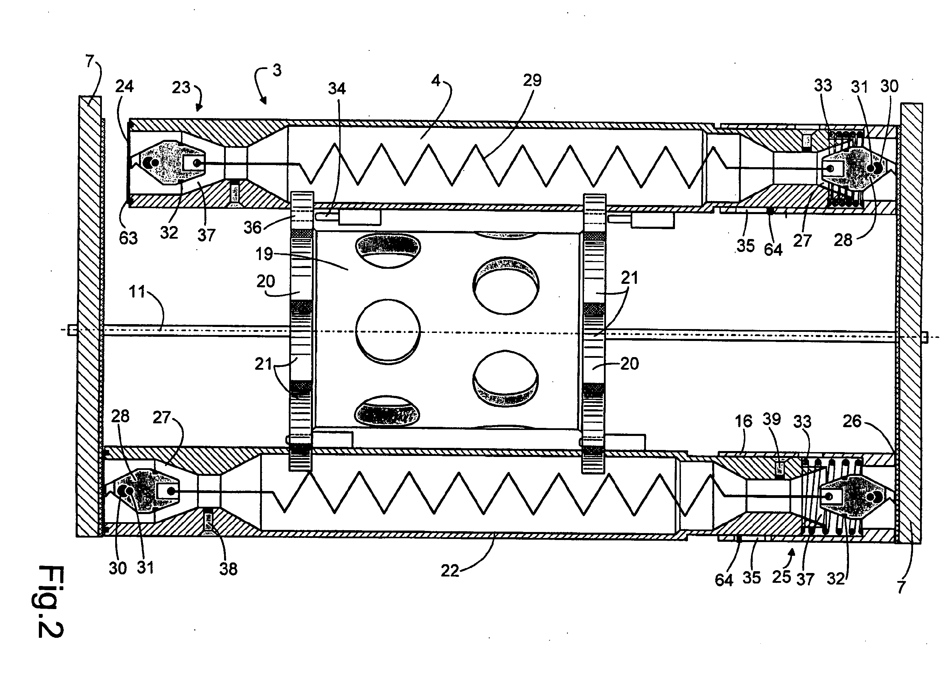

**FIGUR 2** zeigt ein Trommelmagazin **3** mit Probenbehältern **4** in verschiedenen Zuständen in einer Seitenansicht. Das Trommelmagazin **3** ist zwischen den Stirnplatten **7** des Montagegestells **2** auf der zentralen Welle **11** drehbar gelagert und weist einen Trommelkörper **19** mit Endplatten **20** auf. Die Endplatten **20** sind zur sicheren Aufnahme der zylindrischen Probenbehälter **4** mit symmetrisch am Umfang verteilten kreisabschnittförmigen Aufnahmen **21** versehen. Der oben dargestellte Probenbehälter **4** ist in einem Zustand vor seiner Verriegelung in der Vorrichtung zur Wasserprobennahme **1**, der unten dargestellte Probenbehälter **4** befindet sich in fertig verriegeltem Zustand. Jeder Probenbehälter **4** weist ein zylindrisches Probengefäß **22**, ein Einlassventil **23** und einen Gleitring **63** aus PTFE an seiner Einlassseite **24**, ein Auslassventil **25** und eine verschiebbar gelagerte federbelastete Hülse **16** an seiner Auslassseite **26** auf. Das Einlassventil **23** und das Auslassventil **25** werden jeweils durch eine konische Öffnung **27** im Probengefäß **22** und einen Verschlusskegel **28** gebildet. Die Verschlusskegel **28** passen formschlüssig in die konischen Öffnungen **27**. Beide Verschlusskegel **28** sind durch eine Zugfeder **29** verbunden und werden in geöffneter Position der Einlass- und Auslassventile **23,25** mit konkaven Lagerschalen **30** auf in den Probenbehältern **4** fixierten Zylinderstiften **31** gehalten. O-Ringe **32** in den Verschlusskegeln **28** dichten sie im geschlossenen Zustand der Einlass- und Auslassventile **23,25** sicher gegen die zugehörigen konischen Öffnungen **27** ab. Die verschiebbar gelagerte Hülse **16** wird im zusammengeschobenen Zustand durch die Bajonettverriegelung **17** in einer verriegelten Position gegen eine Rückstellfeder **33** gehalten. Dazu wird die Hülse **16** zurückgeschoben und dann rechtwinklig dazu gedreht, so dass ein im Probengefäß **22** fixierter Führungsstift **64** in dem Labyrinth **35** der Bajonettverriegelung **17** in einen am Umfang der Hülse **16** liegenden Gang geführt wird und die Hülse **16** gegen ein Zurückschieben durch die Rückstellfeder **33** sichert. Als Verdrehsicherung weisen die Probenbehälter **4** vier zylindrische Passstifte **34**, die paarweise in zugehörige Passbohrungen **36** der Endplatten **20** des Trommelkörpers **19** eingeführt werden können, als formschlüssige Steckverbindung auf. Zum Einsatz des Probenbehälters **4** wird die Hülse **16** gegen die Kraft der Rückstellfeder **33** in die verriegelte Stellung gebracht. Der Probenbehälter **4** wird nun derart in eine der kreissegmentförmigen Aufnahmen **21** in den Endplatten **20** des Trommelkörpers **19** eingelegt, dass die Passstifte **34** direkt vor ihren zugehörigen Passbohrungen **36** liegen. Durch horizontales Verschieben des Probenbehälters **4** werden die Passstifte **34** in die Passbohrungen **36** eingeführt und fixieren so den Probenbehälter **4** in seiner Endposition. Anschließend wird die Hülse **16** zurückgedreht, so dass der Führungsstift **64** im Labyrinth **35** der Bajonettverriegelung **17** in einen axial angeordneten Gang geführt wird und damit die Hülse **16** freigegeben wird. Die Rückstellfeder **33** schiebt nun die Hülse **16** axial soweit heraus, bis sie gegen den PTFE-Belag **18** der zugehörigen Stirnplatte **7** des Montagegestells **2** stößt und damit den Probenbehälter **4** in einer kraftschlüssigen Einspannverbindung hält. Nach dem fertigen Einsetzen der Probenbehälter **4** in das Trommelmagazin **3** sind die Einlass- und Auslassventile **23,25** geöffnet und bilden symmetrische Ringspalte **37** zum Ein- und Ausströmen des Probenwassers. Schließlich weisen die Probenbehälter **4** noch je eine Ablassöffnung **38** und eine Belüftungsöffnung **39**, die beide durch Schrauben abgedichtet sind, zum Ablassen der gesammelten Wasserprobe auf.

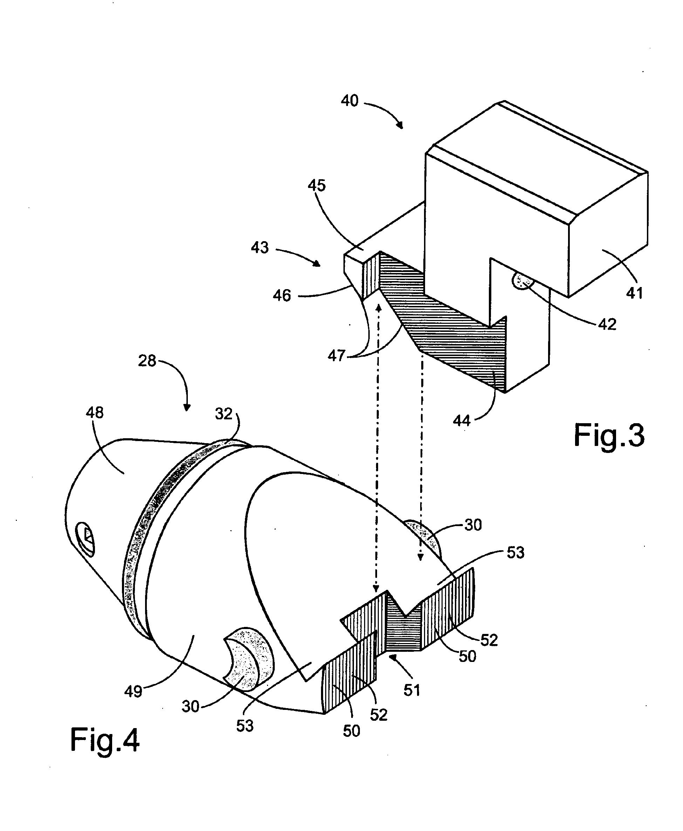

**FIGUR 3** zeigt einen Auslösehebel **40** in perspektivischer Ansicht. Die Auslösehebel **40** dienen dem Verschluss der Probenbehälter **4** nach der Wasserprobennahme. Sie weisen einen Führungskörper **41** mit einer Führungsbohrung **42** und einen einstückig damit verbundenen und in Richtung auf den Probenbehälter **4** vorstehenden Mitnehmer **43** aus einem abgeflachten Träger **44** und einer beidseitig symmetrisch überstehenden Nase **45** auf. Die Vorderkante **46** des Mitnehmers **43** ist als ebene Einlaufschräge **47** ausgebildet.

**FIGUR 4** zeigt einen Verschlusskegel **28** in perspektivischer Ansicht. Die Verschlusskegel **28** weisen einen kegelförmigen Verschlussabschnitt **48** mit dem O-Ring **32** und einen zylindrischen Mitnehmerabschnitt **49** auf. Dieser ist mit zwei Mitnehmerhaken **50** mit einer dazwischen angeordneten rechteckigen Ausnehmung **51** ausgebildet. Die Mitnehmerhaken **50** weisen je eine parallel zur Stirnseite des Probenbehälters **4** ausgerichtete Prallfläche **52** auf und bilden mit dem übrigen Mitnehmerabschnitt **49** eine Hinterschneidung **53**. An beiden Seiten des Mitnehmerabschnitts **49** sind die konkaven Lagerschalen **30** zur Lagerung des Verschlusskegels **28** auf in den Probenbehältern **4** fixierten Zylinderstiften **31** angeordnet.

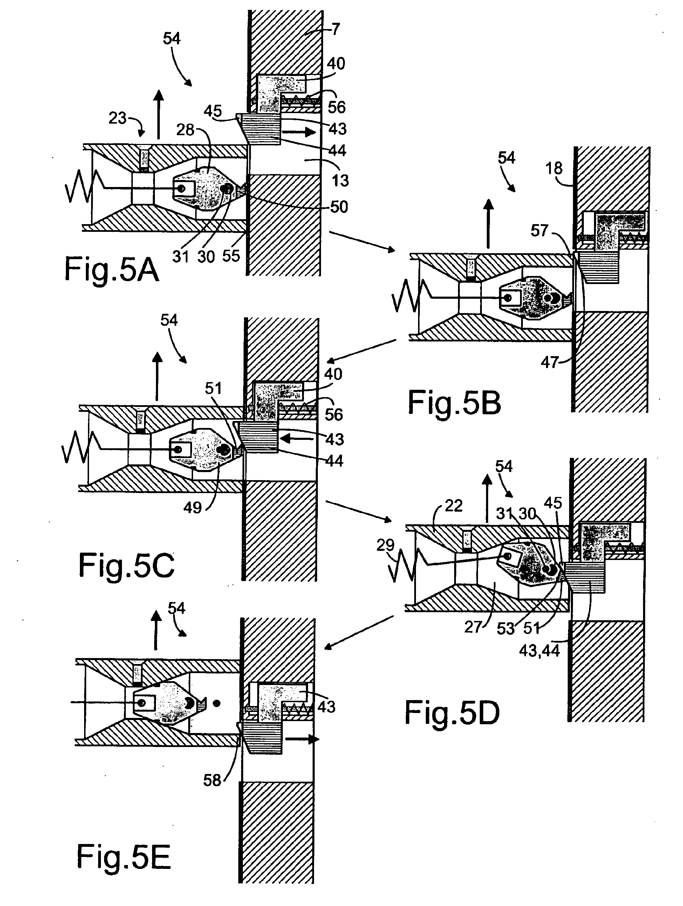

**FIGUR 5A-E** zeigt eine Betätigungsvorrichtung **54** mit einem Ablauf in fünf Stellungen in Seitenansicht. Die Betätigungseinrichtung **54** dient dem Verschließen der Probenbehälter **4** nach der Wasserprobennahme und wird automatisch ausgelöst durch die Drehung des Trommelmagazins **3** durch die Anwahleinrichtung **5**, hier angedeutet durch die senkrechten Pfeile über dem abgeschnitten dargestellten Probenbehälter **4**. In **Figur 5A** beginnt die Stirnseite **55** des Probenbehälters **4** die Einlassöffnung **13** in der Stirnplatte **7** des Montaggestells **2** zu überdecken. Der Verschlusskegel **28** ist in geöffneter Position des Einlassventils **23** mit seinem Lagerschalen **30** auf den Zylinderstiften **31** gehalten, seine Mitnehmerhaken **50** stehen um höchstens 0,5 mm hinter die Stirnseite **55** des Probenbehälters **4** zurück. Der Auslösehebel **40** befindet sich in einer durch eine Feder **56** bestimmten ersten Endlage, wobei die Nase **45** und zumindest 1 mm des Trägers **44** des Mitnehmers **43** aus der Stirnplatte **7** hervorstehenden. In **Figur 5B** hat die vordere Kante **57** der Stirnseite **55** des Probenbehälters **4** den Auslösehebel **40** mit seiner Einlaufschräge **47** in eine mit dem PTFE-Belag **18** der Stirnplatte **7** bündige zweite Endlage zurück geschoben. In **Figur 5C** steht der Probenbehälter **4** mit seinen geöffneten Einlass- und Auslassventilen **23,25** direkt vor der Einlassöffnung **13** der Stirnplatte **7** des Montagegestells **2**. An dieser Stelle stoppt die Anwahleinrichtung **5** die Drehung des Trommelmagazins **3** und ermöglicht die Wasserprobennahme. Der Auslösehebel **40** ist dabei von der vordere Kante **57** der Stirnseite **55** des Probenbehälters **4** freigegeben worden und durch die Kraft der Feder **56** wieder in seine erste Endlage zurückgefallen. Der Träger **44** des Mitnehmers **43** steht nun unmittelbar vor der rechteckigen Ausnehmung **51** des Mitnehmerabschnitts **49** des Verschlusskegels **28**. In **Figur 5D** ist die Wasserprobennahme beendet und Anwahleinrichtung **5** setzt die Drehung des Trommelmagazins **3** fort, um den nächsten Probenbehälter **4** vor die Einlassöffnung **13** zu befördern. Der Träger **44** des Mitnehmers **43** bewegt sich dabei durch die rechteckigen Ausnehmung **51** des Mitnehmerabschnitts **49** des Verschlusskegels **28**, bis die Nase **45** des Mitnehmers **43** in die Hinterschneidung **53** des Verschlusskegels **28** eingreift und diesen mitnimmt. Durch das Verhaken des Mitnehmers **43** und des Verschlusskegels **28** kann der Mitnehmer **43** unter den auftretenden Kräften durch den Verschlusskegel 28 nicht in seine zweite Endlage zurückgedrückt werden. Die Mitnahme des Verschlusskegels **28** bewirkt dessen zunehmende Kippstellung, solange, bis die konkaven Lagerschalen **30** von den in den Probenbehältern **4** fixierten Zylinderstiften **31** abrutschen. Sobald die Verschlusskegel nicht mehr von den Zylinderstiften **31** in geöffneter Stellung der Einlass- und Auslassventile **23,25** gehalten werden, zieht die verbindende Zugfeder **29** die Verschlusskegel **28** in ihre konischen Öffnungen **27** im Probengefäß **22** und dichtet so den Probenbehälter sicher ab. In **Figur 5E** ist dieser Zustand erreicht. Der Probenbehälter **4** wird weiter gedreht und die hintere Kante **58** der Stirnseite **55** des Probenbehälters **4** schiebt nun den Mitnehmer **43** wieder in Richtung auf seine zweite Endstellung zurück.

**FIGUR 6A-C** zeigt eine Strömungsfahne **59** mit einem Ablauf in drei Stellungen in Seitenansicht. Die Strömungsfahne **59** dient dem Zweck, einen korrekt mit Probenwasser gefüllten Probenbehälter **4** anzuzeigen. Für die Wasserprobennahme werden die Probenbehälter **4** mit geöffneten Einlass- und Auslassventilen **23,25** in das Trommelmagazin **3** eingesetzt. Dazu werden die Verschlusskegel **28** aus den konischen Öffnungen **27** gegen die Kraft der sie verbindenden Zugfeder **29** herausgezogen und durch Aufsetzen der konkaven Lagerschalen **30** auf die in der beweglichen Hülse **16** fixierten Zylinderstifte **31** in ihrer Position gehalten. Bei Erschütterungen der Vorrichtung zur Wasserprobennahme **1** kann es vorkommen, dass einzelne Verschlusskegel **28** ungewollt von ihren Positionen entfernt und der zugehörige Probenbehälter verschlossen wird. Es ist dann keine Wasserprobennahme mehr möglich und das in den gefluteten Probenbehältern **4** vorhandene Wasser stellt keine korrekte Wasserprobe dar. Die Strömungsfahne **59** kann nur umklappen, wenn der Probenbehälter **4** von Wasser durchströmt und in einer Position zur Wasserprobennahme vor der Auslassöffnung **15** der Stirnplatten **7** des Montagegestells **2** ist. Wird keine umgeklappte Strömungsfahne **59** bei der Entnahme der Probenbehälter **4** vorgefunden, enthält der Probenbehälter **4** keine korrekte Wasserprobe. Die Strömungsfahne **59** ist in einer Aussparung **60** der beweglichen Hülse **16** der Auslassseite **26** des Probenbehälters **4** drehbar angeordnet. Ein Anschlag **61** sorgt für eine Begrenzung der Drehbewegung. **Figur 6A** zeigt die Ausgangsstellung der Strömungsfahne **59** beim Einsatz der Probenbehälter **4** in das Trommelmagazin **3**. Die Strömungsfahne **59** ist eingeklappt und wird in dieser Stellung durch die Stirnplatte **7** des Montagegestells **2** gehalten. In **Figur 6B** befindet sich der Probenbehälter **4** in einer Position zur Wasserprobennahme mit seiner Auslassseite **26** vor der Öffnung **27** der Stirnplatte **7** des Montagegestells **2**. In der Öffnung **27** ist ein zusätzlicher Bewegungsraum **62** vorgesehen, der die Drehung der Strömungsfahne **59** ausschließlich an dieser Position ermöglicht. Tritt eine Durchströmung des Probenbehälters **4** ein (hier angedeutet durch drei Pfeile), wird die Strömungsfahne **59** durch den Wasserdruck umgeklappt und in ihrer Bewegung durch den Anschlag **61** begrenzt. In **Figur 6C** wurde das Trommelmagazin **3** weitergedreht. Der Probenbehälter **4** steht wieder vor einem geschlossenen Abschnitt der Stirnplatte **7**, wodurch die Strömungsfahne **59** in ihrer ausgedrehten Position fixiert wird und eine erfolgreiche Wasserprobennahme signalisiert.

### BEZUGSZEICHENLISTE

| | |
|---|---|
| **1** | Vorrichtung zur Wasserprobennahme |
| **2** | Montagegestell |
| **3** | Trommelmagazin |
| **4** | Probenbehälter |
| **5** | Anwahleinrichtung |
| **6** | Steuereinrichtung |
| **7** | Stirnplatte |
| **8** | Motor |
| **9** | Schnecke |
| **10** | Schneckenrad |
| **11** | Welle |
| **12** | Zuleitung |
| **13** | Einlaßöffnung |
| **14** | Ableitung |
| **15** | Auslassöffnung |
| **16** | Hülse |
| **17** | Bajonettverriegelung |
| **18** | PTFE-Belag |
| **19** | Trommelkörper |
| **20** | Endplatte |
| **21** | Aufnahme |
| **22** | Probengefäß |
| **23** | Einlassventil |
| **24** | Einlassseite |
| **25** | Auslassventil |
| **26** | Auslassseite |
| **27** | Öffnung |
| **28** | Verschlusskegel |
| **29** | Zugfeder |
| **30** | Lagerschale |
| **31** | Zylinderstift |
| **32** | O-Ring |
| **33** | Rückstellfeder |
| **34** | Passstift |
| **35** | Labyrinth |
| **36** | Passbohrung |
| **37** | Ringspalt |
| **38** | Ablassöffnung |
| **39** | Belüftungsöffnung |
| **40** | Auslösehebel |
| **41** | Führungskörper |
| **42** | Führungsbohrung |
| **43** | Mitnehmer |
| **44** | Träger |
| **45** | Nase |
| **46** | Vorderkante |
| **47** | Einlaufschräge |
| **48** | Verschlussabschnitt |
| **49** | Mitnehmerabschnitt |
| **50** | Mitnehmerhaken |
| **51** | Ausnehmung |
| **52** | Prallfläche |
| **53** | Hinterschneidung. |
| **54** | Betätigungsvorrichtung |
| **55** | Stirnseite |
| **56** | Feder |
| **57** | vordere Kante |
| **58** | hintere Kante |
| **59** | Strömungsfahne |
| **60** | Aussparung |
| **61** | Anschlag |
| **62** | zusätzlicher Bewegungsraum |
| **63** | Gleitring |
| **64** | Führungsstift |

## Patentansprüche

1. Vorrichtung zur Wasserprobennahme (1) mit einem Montagegestell (2), einem Mehrfachmagazin in Form eines Trommelmagazins (3) mit mehreren Probenbehältern (4), die durch individuelle Befestigungseinrichtungen einzeln entnehmbar sind, wobei formschlüssige Steckverbindungen zur Befestigung der Probenbehälter (4) mit dem Trommelmagazin (3) vorgesehen sind, und an einer ersten Stirnseite ein Einlassventil (23) und an einer zweiten Stirnseite ein Auslassventil (25) aufweisen, wobei axial angeordnete konische Öffnungen (27) in den Stirnseiten der Probenbehälter (4) und dazu formschlüssige Verschlusskegel (28) als Einlass- und Auslassventile (23,25) vorgesehen sind, mit einer Betätigungsvorrichtung (54) zum Verschließen der Einlass- und Auslassventile (23,25) und einem Motor (8) mit Getriebe als Auswahleinrichtung (5) für den nächsten zur Wasserprobennahme bestimmten Probenbehälter (4),
**gekennzeichnet durch**
- zwei auf einer Welle (11) als zentraler Längsachse des Montagegestells (2) parallel zueinander angeordnete Stirnplatten (7), zwischen denen das Trommelmagazin (3) horizontal drehbar gelagert ist, wobei die eine Stirnplatte (7) eine Einlassöffnung (13) und die andere Stirnplatte (7) eine Auslassöffnung (15) aufweist, zwischen die ein Probenbehälter (4) im Trommelmagazin (3) eindrehbar ist,
- kraftschlüssige Einspannverbindungen zur Befestigung der Probenbehälter (4) zwischen den Stirnplatten (7) des Montagegestells (2),
- axial zu den Probenbehältern (4) verschiebbare Verschlusskegel (28), die in geöffnetem Zustand symmetrische Ringspalte (37) ausbilden,
- Mitnehmerhaken (50) an den Verschlusskegeln (28) und Auslösehebel (40) in den Stirnplatten (7) des Montagegestells (2) als Betätigungsvorrichtung für die Einlass- und Auslassventile (23,25) und
- eine Anordnung des Motors (8) mit Getriebe zwischen dem Montagegestell (2) und dem Trommelmagazin (3) als Auswahleinrichtung (5) zur Drehung des Trommelmagazins (3) mit den Probenbehältern (4).

2. Vorrichtung zur Wasserprobennahme (1) nach Anspruch 1,
**gekennzeichnet durch**
zylinderförmig ausgebildete Probenbehälter (4).

3. Vorrichtung zur Wasserprobennahme (1) nach Anspruch 1,
**gekennzeichnet durch**
zumindest je eine Passbohrung (36) an Endplatten (20) des Trommelmagazins (3) und zumindest einen, jeder Passbohrung (36) zugeordneten Passstift (34) an den Probenbehältern (4) als formschlüssige Steckverbindungen der Befestigungseinrichtung der Probenbehälter (4) mit dem Trommelmagazin (3).

4. Vorrichtung zur Wasserprobennahme (1) nach Anspruch 1,
**gekennzeichnet durch**
eine an der Auslassseite (26) der Probenbehälter (4) verschiebbar gelagerte federbelastete Hülse (16) mit Bajonettverriegelung (17) als kraftschlüssige Einspannverbindung der Befestigungseinrichtung der Probenbehälter (4) zwischen den Stirnplatten (7) des Montagegestells (2).

5. Vorrichtung zur Wasserprobennahme (1) nach Anspruch 4,
**gekennzeichnet durch**
Polyvinylchlorid als Material der druckfederbelasteten Hülse (16).

6. Vorrichtung zur Wasserprobennahme (1) nach Anspruch 1,
**gekennzeichnet durch**
Polytetrafluorethylen als Beschichtungsmaterial der Stirnplatten (7) des Montagegestells (2).

7. Vorrichtung zur Wasserprobennahme (1) nach Anspruch 1,
**gekennzeichnet durch**
Probenbehälter (4), die an der Einlassseite (24) eine umlaufende Nut mit einem um 0,1 bis 2 mm hervorstehend eingelegten Gleitring (63) aus Polytetrafluorethylen aufweisen.

8. Vorrichtung zur Wasserprobennahme (1) nach den Ansprüchen 2 und 3,
**gekennzeichnet durch**
Endplatten (20) des Trommelmagazins (3) mit kreisabschnittförmigen Aufnahmen (21) in symmetrisch am Umfang verteilter Anordnung am Außenrand zur Aufnahme der zylinderförmigen Probenbehälter (4).

9. Vorrichtung zur Wasserprobennahme (1) nach Anspruch 1,
**gekennzeichnet durch**
Mitnehmerhaken (50) an den Verschlusskegeln (28) mit bündig zu den Stirnseiten der Probenbehälter (4) ausgerichteten Flächen.

10. Vorrichtung zur Wasserprobennahme (1) nach Anspruch 1,
**gekennzeichnet durch**
Federn (56) an den Auslösehebeln (40) in den Stirnplatten (7) des Montagegestells (2), die die Auslösehebel (40) in einer ersten Endlage zumindest bündig, bevorzugt aber hinter den Oberflächen der Stirnplatten (7) zurückstehend und in einer zweiten Endlage um zumindest 1 mm aus den Stirnplatten (7) hervorstehend halten.

11. Vorrichtung zur Wasserprobennahme (1) nach Anspruch 1,
**gekennzeichnet durch**
Probenbehälter (4), die in der federbelasteten Hülse (16) der Auslassseite (26) eine zwischen zwei Endstellungen umklappbare Strömungsfahne (59) aufweisen.

12. Vorrichtung zur Wasserprobennahme (1) nach Anspruch 1,
**gekennzeichnet durch**
Polyvinylidenfluorid als Material der Probenbehälter (4).

13. Vorrichtung zur Wasserprobennahme (1) nach Anspruch 1,
**gekennzeichnet durch**
Polyethylen als Material des Montagegestells (2).

14. Vorrichtung zur Wasserprobennahme (1) nach Anspruch 1,
**gekennzeichnet durch**
eine Verbindung der Verschlusskegel (28) der Einlass- und Auslassventile (23,25) **durch** eine im Inneren der Probenbehälter (4) verlaufende Zugfeder (29).

15. Vorrichtung zur Wasserprobennah me (1) nach Anspruch 1,
**gekennzeichnet durch**
eine Lagerung der Verschlusskegel (2E) mit konkaven Lagerschalen (30) auf in den Probenbehältern (4) fixierten Zylinderstiften (31) in geöffnetem Zustand der Einlass- und Auslassventile (23,25)

16. Vorrichtung zur Wasserprobennahme (1) nach Anspruch 1,
**gekennzeichnet durch**
eine Weiterdrehbarkeit des Trommelmagazins (3) um jeweils eine **durch** die symmetrisch am Umfang verteilte Anordnung der Probenbehälter (4) bestimmte Teilung bei jeder Betätigung **durch** die Anwahleinrichtung (5).

## Claims

1. A device for taking water samples (1), having a mounting frame (2), a multi-compartment magazine in the form of a drum magazine (3) with a plurality of sample containers (4), which can be removed individually by individual fastening means, wherein form-fitting plug-in connections are provided to fasten the sample containers (4) to the drum magazine (3), and have an inlet valve (23) on a first end face and an outlet valve (25) on a second end face, wherein axially arranged conical openings (27) are provided in the end faces of the sample containers (4) and closing cones (28) which are form-fitting thereto are provided as inlet and outlet valves (23, 25), having an actuation device (54) for closing the inlet and outlet valves (23, 25) and a motor (8) with a gear mechanism as the selection means (5) for the next sample container (4) defined for taking a water sample,
**characterised by**
- two end plates (7), which are arranged parallel to each other on a shaft (11) as the central longitudinal axis of the mounting frame (2) and between which the drum magazine (3) is mounted such that it can rotate horizontally, wherein one end plate (7) has an inlet opening (13) and the other end plate (7) has an outlet opening (15), between which a sample container (4) in the drum magazine (3) can be rotated in,
- force-fitting clamping connections for fastening the sample container (4) between the end plates (7) of the mounting frame (2),
- closing cones (28) which can be displaced axially to the sample containers (4) and form symmetrical annular gaps (37) in the opened state,
- dog hooks (50) on the closing cones (28) and triggering levers (40) in the end plates (7) of the mounting frame (2) as the actuation device for the inlet and outlet valves (23, 25) and
- an arrangement of the motor (8) with the gear mechanism between the mounting frame (2) and the drum magazine (3) as the selection means (5) for rotating the drum magazine (3) with the sample containers (4).

2. The device for taking water samples (1) according to Claim 1,
**characterised by**
cylindrical sample containers (4).

3. The device for taking water samples (1) according to Claim 1,
**characterised by**
at least one dowel hole (36) in each case on end plates (20) of the drum magazine (3) and at least one dowel pin (34), which is assigned to each dowel hole (36), on the sample containers (4) as the form-fitting plug-in connections of the means of fastening the sample containers (4) to the drum magazine (3).

4. The device for taking water samples (1) according to Claim 1,
**characterised by**
a spring-loaded sleeve (16), which is mounted in a displaceable manner on the outlet side (26) of the sample container (4), having a bayonet lock (17) as the force-fitting clamping connection of the means of fastening the sample container (4) between the end plates (7) of the mounting frame (2).

5. The device for taking water samples (1) according to Claim 4,
**characterised by**
polyvinyl chloride as the material of the compressive-spring-loaded sleeve (16).

6. The device for taking water samples (1) according to Claim 1,
**characterised by**
polytetrafluoroethylene as the coating material of the end plates (7) of the mounting frame (2).

7. The device for taking water samples (1) according to Claim 1,
**characterised by**
sample containers (4), which have a circumferential groove on the inlet side (24), which has a sliding ring (63) which is inserted such that it projects by 0.1 to 2 mm and consists of polytetrafluoroethylene.

8. The device for taking water samples (1) according to Claims 2 and 3.
**characterised by**
end plates (20) of the drum magazine (3) having circular-segment-shaped receptacles (21) in an arrangement distributed symmetrically around the circumference at the outer edge of the cylindrical sample container (4).

9. The device for taking water samples (1) according to Claim 1,
**characterised by**
dog hooks (50) on the closing cones (28) with faces which are aligned flush with the end faces of the sample containers (4).

10. The device for taking water samples (1) according to Claim 1,
**characterised by**
springs (56) on the triggering levers (40) in the end plates (7) of the mounting frame (2), which hold the triggering levers (40) in a first end position such that they are retracted at least flush with but preferably behind the surfaces of the end plates (7) and in a second end position such that they project at least 1 mm out of the end plates (7).

11. The device for taking water samples (1) according to Claim 1,
**characterised by**
sample containers (4), which have a flow flag (59), which can be switched between two end positions, in the spring-loaded sleeve (16) of the outlet side (26).

12. The device for taking water samples (1) according to Claim 1,
**characterised by**
polyvinyl fluoride as the material of the sample container (4).

13. The device for taking water samples (1) according to Claim 1,
**characterised by**
polyethylene as the material of the mounting frame (2).

14. The device for taking water samples (1) according to Claim 1,
**characterised by**
a connection of the closing cones (28) of the inlet and outlet valves (23, 25) by means of a tensile spring (29) running in the interior of the sample container (4).

15. The device for taking water samples (1) according to Claim 1,
**characterised by**
bearing of the closing cones (28) with concave bearing cups (30) on cylindrical pins (31) fixed in the sample containers (4) in the open state of the inlet and outlet valves (23, 25).

16. The device for taking water samples (1) according to Claim 1,
**characterised by**
an ability of the drum magazine (3) to rotate further by in each case one division defined by the arrangement of the sample containers (4) distributed symmetrically at the circumference on each actuation by the selection means (5).

## Revendications

1. Dispositif pour le prélèvement d'échantillons d'eau (1) avec une structure de montage (2), un magasin à plusieurs compartiments sous la forme d'un magasin à tambour (3) avec plusieurs récipients à échantillon (4) pouvant être retirés individuellement par des dispositifs de fixation individuels, dans lequel il est prévu des assemblages par emboîtement de forme permettant de fixer les récipients à échantillon (4) au magasin à tambour (3) et comportant une soupape d'admission (23) sur un premier côté frontal ainsi qu'une soupape d'évacuation (25) sur un deuxième côté frontal, dans lequel il est prévu des ouvertures coniques (27) agencées axialement dans les côtés frontaux des récipients à échantillon (4) ainsi que des cônes de fermeture (28) de forme complémentaire, en tant que soupapes d'admission et d'évacuation (23, 25), avec un dispositif d'actionnement (54) pour la fermeture des soupapes d'admission et d'évacuation (23, 25) et un moteur (8) avec engrenage en tant que dispositif de sélection (5) pour le récipient à échantillon (4) destiné au prélèvement d'échantillon suivant,
**caractérisé en ce que**
- deux plaques frontales (7) agencées parallèlement l'une par rapport à l'autre sur un arbre (11) en tant qu'axe longitudinal médian de la structure de montage (2), entre lesquelles le magasin à tambour (3) est monté de façon à pouvoir tourner horizontalement, où l'une des plaques frontales (7) comporte une ouverture d'admission (13) et l'autre plaque frontale (7) comporte une ouverture d'évacuation (15), entre lesquelles un récipient à échantillon (4) peut être vissé dans le magasin à tambour (3),
- des assemblages de serrage à force pour la fixation des récipients à échantillon (4) entre les plaques frontales (7) de la structure de montage (2),
- des cônes de fermeture (28) déplaçables axialement par rapport aux récipients à échantillon (4) et formant une fente annulaire symétrique (37) dans l'état ouvert,
- des crochets d'entraînement (50) sur les cônes de fermeture (28) et des leviers de déclenchement (40) dans les plaques frontales (7) de la structure de montage (2), en tant que dispositif d'actionnement pour les soupapes d'admission et d'évacuation (23, 25), et
- un agencement du moteur (8) avec engrenage entre la structure de montage (2) et le magasin à tambour (3), en tant que dispositif de sélection (5) pour la rotation du magasin à tambour (3) avec les récipients à échantillon (4).

2. Dispositif pour le prélèvement d'échantillons d'eau (1) selon la revendication 1,
**caractérisé par**
des récipients à échantillon (4) conçus en forme de cylindres.

3. Dispositif pour le prélèvement d'échantillons d'eau (1) selon la revendication 1,
**caractérisé par**
au moins un alésage d'ajustement (36) dans chacune des plaques terminales (20) du magasin à tambour (3), et au moins une tige d'ajustement (34) attribuée à chacun des alésages d'ajustement (36), sur les récipients à échantillon (4), en tant qu'assemblages par emboîtement de forme du dispositif de fixation des récipients à échantillon (4) au magasin à tambour (3).

4. Dispositif pour le prélèvement d'échantillons d'eau (1) selon la revendication 1,
**caractérisé par**
une douille (16) contrainte par ressort, agencée de façon coulissante du côté de l'évacuation (26) des récipients à échantillon (4), avec un verrouillage à baïonnette (17) en tant qu'assemblage de serrage à force du dispositif de fixation des récipients à échantillon (4) entre les plaques frontales (7) de la structure de montage (2).

5. Dispositif pour le prélèvement d'échantillons d'eau (1) selon la revendication 4,
**caractérisé par**
du polychlorure de vinyle en tant que matériau de la douille contrainte par ressort (16).

6. Dispositif pour le prélèvement d'échantillons d'eau (1) selon la revendication 1,
**caractérisé en ce que**
du poly-tétra-fluor-éthylène en tant que revêtement des plaques frontales (7) de la structure de montage (2).

7. Dispositif pour le prélèvement d'échantillons d'eau (1) selon la revendication 1,
**caractérisé par**
des récipients à échantillon (4) comportant une rainure périphérique avec, côté admission (24), une bague coulissante (63) insérée de façon à faire saillie de 0,1 à 2 mm, constituée de poly-tétra-fluor-éthylène.

8. Dispositif pour le prélèvement d'échantillons d'eau (1) selon les revendications 2 et 3,
**caractérisé par**
des plaques terminales (20) du magasin à tambour (3), avec des admissions (21) en forme de segment de cercle dans un agencement symétrique périphérique sur le bord extérieur, pour l'admission des récipients à échantillon (4) cylindriques.

9. Dispositif pour le prélèvement d'échantillons d'eau (1) selon la revendication 1,
**caractérisé par**
des crochets d'entraînement (50) sur les cônes de fermeture (28), avec des surfaces orientées en affleurement avec les faces frontales des récipients à échantillon (4).

10. Dispositif pour le prélèvement d'échantillons d'eau (1) selon la revendication 1,
**caractérisé par**
des ressorts (56) sur les leviers de déclenchement (40) dans les plaques frontales (7) de la structure de montage (2), permettant de maintenir les leviers de déclenchement (40) au moins en affleurement avec les surfaces supérieures des plaques frontales (7), mais de préférence derrière celles-ci, dans une première position finale, et en saillie d'au moins 1 mm hors des plaques frontales (7) dans une deuxième position finale.

11. Dispositif pour le prélèvement d'échantillons d'eau (1) selon la revendication 1,
**caractérisé par**
des récipients à échantillon (4) comportant un ergot d'écoulement (59) rabattable entre deux positions finales, situé dans la douille contrainte par ressort (16) du côté évacuation (26).

12. Dispositif pour le prélèvement d'échantillons d'eau (1) selon la revendication 1,
**caractérisé par**
du fluorure de polyvinylidène en tant que matériau des récipients à échantillon (4).

13. Dispositif pour le prélèvement d'échantillons d'eau (1) selon la revendication 1,
**caractérisé par**
du polyéthylène en tant que matériau de la structure de montage (2).

14. Dispositif pour le prélèvement d'échantillons d'eau (1) selon la revendication 1,
**caractérisé par**
un assemblage des cônes de fermeture (28) des soupapes d'admission et d'évacuation (23, 25) par un ressort de traction (29) s'étendant à l'intérieur des récipients à échantillon (4).

15. Dispositif pour le prélèvement d'échantillons d'eau (1) selon la revendication 1,
**caractérisé par**
un palier pour les cônes de fermeture (28), avec des coupelles de support concaves (30) montées sur des goupilles cylindriques (31) fixées dans les récipients à échantillon (4), dans l'état ouvert des soupapes d'admission et d'évacuation (23, 25).

16. Dispositif pour le prélèvement d'échantillons d'eau (1) selon la revendication 1,
**caractérisé par**
la possibilité de poursuivre la rotation du magasin à tambour (3), respectivement sur une distance déterminée par l'agencement des récipients à échantillon (4) répartis symétriquement sur le pourtour, à chaque actionnement par le dispositif de sélection (5).
